# EUROPÄISCHE PATENTANMELDUNG

(11) **EP 1 016 730 A1**
(43) Veröffentlichungstag der Anmeldung: **05.07.2000**
(21) Anmeldenummer: 99126239.5
(22) Anmeldetag: 30.12.1999
(51) Int. Cl.: C12Q 1/68

(54) **Gentest zur Bestimmung des Proliferationsgrades der Brust**

(30) Priorität: 30.12.1998 DE 19860536
(71) Anmelder: HESCH, Rolf Dieter, Prof. Dr., D-78464 Konstanz (DE)
(72) Erfinder: HESCH, Rolf Dieter, Prof. Dr., D-78464 Konstanz (DE)
(74) Vertreter: Forstmeyer, Dietmar, Dr. rer. nat., Dipl.-Chem.

(57) **Zusammenfassung**

Die vorliegende Erfindung betrifft Verfahren zur in vitro Diagnose des Proliferationsgrades der weiblichen Brust, das dadurch gekennzeichnet ist, daß die Konzentration an zirkulierenden brustdrüsenspezifischen DNA-Sequenzen im Blutplasma der Frau bestimmt wird.

## Beschreibung

Die vorliegende Erfindung betrifft ein Verfahren zur in vitro Diagnose des Proliferationsgrades der weiblichen Brust, wobei die Konzentration an zirkulierenden brustdrüsenspezifischen DNA-Sequenzen im Blutplasma der Frau bestimmt wird.

Die Morbidität (Erkrankungsrate) von Brustkrebs steigt seit Jahren kontinuierlich an, während die Kurve über die Sterblichkeit von Brustkrebs zum Stillstand gekommen ist. Die moderne Tumorforschung ist sich darüber einig, daß Krebs allein schon durch vererbte (genomische) Schäden (Mutationen) entstehen kann. Häufiger kommen allerdings im Laufe des Lebens zu einer vererbten Mutation weitere somatische Mutationen. In der Zwischenzeit gibt es zahlreiche Untersuchungen aus europäischen Ländern (z.B. Italien und Holland) die zeigen, daß die Rate an Brustkrebs bei Frauen mit regelmäßigem Menstruationszyklus deutlich höher ist als bei Frauen, die einen irregulären Zyklus haben. Es konnte auch gezeigt werden, daß die Morbidität an Brustkrebs statistisch signifikant auch nach Korrektur für andere Faktoren, mit der Anzahl der im Leben durchgemachten Menstruationszyklen korreliert. Seit langem wird diskutiert, ob die ansteigende Krankheitskurve für Brustkrebs durch kombinierte orale Kontrazeptiva (Verhütungspillen) respektive durch den Hormonersatz der klimakterischen und menopausalen Frau verursacht wird. Eine 1996 im Lancet publizierte kollaborative Reanalyse von mehr als 53.000 Frauen mit Brustkrebs und 100.000 Frauen ohne Brustkrebs aus 54 epidemiologischen Studien in 25 Ländern ergab zwei wesentliche Schlußfolgerungen:

Während der Behandlung mit oralen Kontrazeptiva und 10 Jahre danach sieht man ein leicht ansteigendes Risiko für Brustkrebs. Frauen die gegenwärtig Kontrazeptiva nehmen, haben ein Risiko von 1,24; 1 - 4 Jahre nach Beendigung der Medikation beträgt das Risiko 1,16 und 5-9 Jahre nach Beendigung der kontrazeptiven Therapie 1,07. 10 Jahre nach Beendigung einer kontrazeptiven Medikation ist das Risiko etwa 1.

Insgesamt wurde daraus der Schluß gezogen, daß Kontrazeptiva das Brustkrebsrisiko nicht wesentlich erhöhen, es gibt aber Subgruppen von Frauen, bei denen ein deutliches Risiko vorhanden ist, welches durch die statistische Analyse von großen Gruppen verschwindet. Aus der gleichen Untersuchungsgruppe gibt es eine entsprechende Untersuchung zum Hormonersatz (Hormonsubstitution). Das Ergebnis zeigt eindeutig, daß mit zunehmender Verwendung eines gegenwärtig gebräuchlichen Hormonersatzpräparates (einer Kombination aus Östrogen und Gestagen) die Brustkrebsrate ansteigen kann.

Bedeutend ist das Ergebnis einer Untersuchung von Pike (Mann, R.D., Hrsg., Oral Contraceptives and Breast Cancer, Lancaster, England: Parthenon Publ. Group, 1990; 323-48) worin gezeigt wird, daß bei Frauen, bei welchen um das Alter von 30 Jahren eine beidseitige Ovarektomie durchgeführt wurde, das Mammakarzinomrisiko fast um die Hälfte niedriger liegt als bei Frauen mit intakten Eierstöcken. Ursprünglich haben die Autoren ihre Befunde dahingehend interpretiert, daß der Wegfall der ovariellen Östrogenproduktion das Mammakarzinomrisiko vermindere. Die Ergebnisse lassen sich aber genauso dahingehend interpretieren, daß durch Wegfall der zyklischen Hormonstimulation durch Östrogen/Gestagen in jedem Menstruationszyklus die Brust stimuliert wird.

Aus dem vorgesagten ist geschlossen worden, daß es sowohl beim natürlich ablaufendem Menstruationszyklus, bei der zyklisch durchgeführten kombinierten oralen Kontrazeption und bei der zyklisch durchgeführten Hormonsubstitution mit Östrogenen und Gestagenen jeden Monat in der zweiten Zyklushälfte für etwa 12 Tage zu einer Stimulation von Mitose und Proliferation im Brustdrüsengewebe kommt. Es kann gezeigt werden, daß Östrogene das Brustdrüsengewebe alleine in Abhängigkeit von der jeweiligen monatlichen Östrogenproduktion in unterschiedlichem Umfang in der ersten Zyklushälfte stumilieren können, und daß diese Stimulation durch die nachfolgende Ausschüttung von Gelbkörperhormon in der zweiten Zyklushälfte intensiviert wird. Es liegen Belege dafür vor, daß diese alle vier Wochen erfolgende sequentielle Stimulation des Brustdrüsengewebes sowohl bei der normal menstruierenden Frau, als auch bei der Frau unter oraler kombinierter Kontrazeption aber auch beim zyklischem Hormonersatz zu einer Stimulation von Mitose und Proliferation im Brustdrüsengewebe führt. Die wissenschaftliche Literatur zeigt, daß in mitotischproliferativ stimuliertem Gewebe somatische Mutationen häufiger vorkommen als in ruhendem Gewebe. Durch diese "Öffnung des Fensters mit der höchsten Mutabilität" kommt es jeden Monat durch die kombinierte hormonale Stimulation zu vermehrten somatischen Genschäden im Brustdrüsengewebe. Je intensiver die hormonale Stimulation sowohl durch hohe endogene Hormonproduktion aber auch durch exogene Hormonzufuhr bei Kontrazeption und Hormonersatz ist, desto mehr werden im Laufe des Lebens kumulativ Mutationen (Genschäden) angehäuft. In Naturvölkern, wo Brustkrebs relativ selten beobachtet wird, haben Frauen in ihrem Leben maximal 40-50 Menstruationszyklen. In unseren heutigen westlichen Kulturen kann eine Frau bis zu 520 Menstruationszyklen im Leben haben.

Dieses Modell erklärt möglicherweise auch die ansteigende Morbidität für Brustkrebs, da mit der monatlichen Öffnung des Brustdrüsengewebes durch die hormonale Stimulation Mutationen in das Brustdrüsengewebe hineingetragen werden. Das Ausmaß der Genschäden sowie die Anzahl der Mutationen bestimmen dabei den Zeitpunkt und die Aggressivität des entstehenden Brustkrebses.

Seit 30 Jahren wird eine Horrnonersatzbehandlung bei der klimakterischen und postmenopausalen Frau durchgeführt. Es ergibt sich dabei die Frage, ob durch einen kombinierten zyklischen Hormonersatz bei der klimakterischen und postmenopausalen Frau weiterhin mutationsauslösende Stimulationen in das Brustdrüsengewebe hineingetragen werden können. Dazu liegen zahlreiche Untersuchungen vor, die darauf hindeuten, daß das Brustkrebsrisiko sowohl bei einer ausschließlich erfolgenden Östrogenersatzbehandlung als auch bei der kombinierten zyklischen Ersatzbehandlung mit Östrogenen und Gestagenen ansteigen kann, und jüngste Metaanalysen von großen Studien deuten wiederum darauf hin, daß dies tatsächlich der Fall ist. Vor allen Dingen scheint eine Hormonbehandlung, die länger als 10 Jahre dauert, das Brustkrebsrisiko zu erhöhen.

Es gibt Untersuchungen, die darauf hindeuten, daß die kontinuierliche kombinierte Gabe von Hormonen ohne einen zyklischen Wechsel dann keinen Brustkrebs auslöst, wenn die gewählten Dosen von Östrogen und Gelbkörperhormon so kombiniert werden, daß eine übermäßige mitotische und proliferative Stimulation der Brust unterbleibt. Untersuchungen an Affen haben gezeigt, daß es bei einer überstimulation von Brustdrüsengewebe durch Östrogenkonzentrationen, die dreimal so hoch wie diejenigen sind, die bei einer entsprechenden Substitution beim Menschen erreicht werden, zu einer deutlichen Stimulation der Proliferation von Brustdrüsengewebe kommt. Eine permanente mitotische Proliferationsstimulation des Brustdrüsengewebes öffnet dieses wiederum für die Aufsammlung von somatischen Mutationen.

Ein monatlich 12 Tage anhaltender Zustand der Hyperproliferation von Brustdrüsengewebe kann also sowohl durch menstruelle Hormonausschüttung als auch durch Gabe (oraler) zyklischkombinierter Kontrazeptiva ausgelöst werden. Ein dauerhafter Zustand der Hyperproliferation von Brustdrüsengewebe kann aber auch dadurch hervorgerufen werden, daß die Dosis der ununterbrochen erfolgenden kontinuierlich-kombinierten Gabe von Östrogen und Gestagen bei der Hormonersatzbehandlung zu hoch gewählt wird.

Aufgrund des vorstehend Ausgeführten und unter Verwendung von Fig. 3 aus Am. J. Obstet. Gynecol. 1997, 176, 123-8 der Arbeitsgruppe um Söderquist in Stockholm läßt sich eine "eumitotische Grenze" formulieren; siehe auch Hesch, R.D. und Kenemans, P, Journal of Gynaecology and Obstetics. Unterhalb dieser eumitotischen Grenze kommt es weder im normalen Menstruationszyklus oder durch orale zyklisch-kombinierte Kontrazeptiva, noch durch eine kontinuierliche kombinierte Hormonmedikation zu einer Stimulation des Brustdrüsengewebes.

Oberhalb dieser eumitotischen Grenze findet sich hingegen eine solche mitotische proliferative Stimulation.

Bisher kann man einen solchen Zustand der Hyperproliferation direkt nur durch eine Feinnadelpunktion von Brustdrüsengewebe diagnostizieren, wobei die dabei gewonnenen Zellen mit entsprechenden, die Zeliproliferation anzeigenden Antikörpern dargestellt werden. Neben der Feinnadelaspiration gibt es außerdem indirekte bildgebende Möglichkeiten, den Stimulationsgrad von Brustdrüsengewebe zu bestimmen, nämlich die Mammographie, die Kernspintomographie und Ultraschalluntersuchungen.

Die Feinnadelaspiration stellt zwar eine gute Methode dar, um die eumitotische Grenze zu bestimmen, ist jedoch in der Praxis wegen der für die Patientinnen sehr unangenehmen Punktion nicht als Routinemaßnahme anzuwenden.

Die bildgebenden Verfahren sind wegen der Strahlenbelastung, der ungenügenden metrischen Auswertbarkeit und auch wegen der Kosten der Untersuchung und ihrer Verfügbarkeit nur begrenzt einzusetzen.

Aufgrund des vorstehend Gesagten gibt es also bis jetzt kein eindeutiges, routinemäßig einsetzbares Verfahren, welches die "eumitotische Grenze" am Brustdrüsengewebe unter Hormonwirkung bestimmen läßt.

Es ist daher die Aufgabe der vorliegenden Erfindung, ein routinemäßig einsetzbares Verfahren zur Bestimmung der eumitotischen Grenze bereitzustellen, mit dem die Nachteile des Standes der Technik überwunden werden, und mit dem die Unannehmlichkeiten für die Patientinnen verringert werden.

Diese Aufgabe wird durch ein Verfahren zur Diagnose des Proliferationsgrades der weiblichen Brust gelöst, das dadurch gekennzeichnet ist, daß die Konzentration an zirkulierenden brustdrüsenspezifischen DNA-Sequenzen im Blut, insbesondere im Blutplasma einer Frau bestimmt wird.

Das erfindungsgemäße Verfahren basiert auf den folgenden Feststellungen:

Vor kurzen konnte erstmals gezeigt werden, daß im Plasma frei zirkulierende DNA vorhanden ist. Dies war lange umstritten, da man geglaubt hatte, daß DNA nur in Zellen und Zellkernen gefunden wird, aber nicht frei im Plasma vorhanden ist.

In bisher unveröffentlichten Eigenuntersuchungen konnten wir nun erstmals zeigen, daß die Konzentration an frei zirkulierender DNA in der ersten Zyklushäfte (vom 1. bis 12. Zyklustag) deutlich niedriger ist als in der zweiten Zyklushälfte (vom 15. bis 28. Zyklustag), und somit die Konzentration an frei zirkulierender DNA im Plasma menstruationsabhängig, respektive in Abhängigkeit von einer Östrogen/Gestagen Stimulation variiert. Insbesondere konnte gezeigt werden, daß die Konzentration an frei zirkulierenden brustdrüsenspezifischen DNAs vom 10. zum 22. Tag des Zyklus ansteigt.

Erfindungsgemäß wird es nun also erstmals möglich, den Proliferationsgrad bzw. die eumitotische Grenze oder den Stimulationsgrad von Brustgewebe unter Hormoneinwirkung zu diagnostizieren, ohne auf die problematischen Methoden des Stands der Technik zurückgreifen zu müssen.

Das erfindungsgemäße Verfahren ist insbesondere dadurch gekennzeichnet, daß Blutplasma verwendet wird, das
a) einer normal menstruierenden Frau (d.h. einer Frau, die keine Hormone zuführt),
b) einer Frau, die Kontrazeptiva zuführt, oder
c) einer klimakterischen oder postmenopausalen Frau, die Hormone zuführt,
in der zweiten und gegebenenfalls auch in der ersten Zyklushälfte entnommen wird.

Erfindungsgemäß wird einer Patientin z.B. während der zweiten Zyklushälfte, vorzugsweise am 22. Tag des Zyklus, Blut entnommen und die Konzentration an brustspezifischer DNA in an sich üblicher Weise wie durch Gel-Elektrophorese, Anfärbung mit Ethidiumbromid und anschließendem Konzentrationsvergleich mit einer Verdünnungsreihe einer bekannten DNA-Lösung bestimmt. Ist die Konzentration der DNA besonders hoch, z.B. 5-10 mal so hoch wie an anderen Tagen, findet im Brustgewebe eine zu hohe Stimulation der Mitose statt, d.h. die eumitotische Grenze wird überschritten. Dadurch wird die Gefahr einer Mutation des Brustgewebes erhöht, was langfristig zu Brustkrebs führen kann.

Als besonders vorteilhaft hat es sich erwiesen, die Konzentrationen der brustdrüsenspezifischen DNA-Sequenzen im Blutplasma aus der ersten und zweiten Zyklushälfte zu vergleichen.

Dazu wird einer Patientin zusätzlich während der ersten Zyklushälfte, vorzugsweise am 14. Tag des Zyklus, Blut entnommen, und wiederum, wie vorstehend angegeben, die Konzentration der brustdrüsenspezifischen DNA bestimmt.

Die eumitotische Grenze wird überschritten, wenn das Verhältnis der Konzentrationen der brustdrüsenspezifischen DNA-Sequenzen im Blutplasma aus der ersten und der zweiten Zyklushälfte oberhalb von 1:5, insbesondere oberhalb von 1:10 liegt.

Um das Untersuchungsergebnis für Brustdrüsengewebe spezifisch zu machen, können neben dem direkten DNA-Nachweis im Plasma z.B. die Polymerase-Kettenreaktion (PCR) oder andere, an sich übliche Amplifikationsverfahren verwendet werden, um in der Plasma-DNA brustdrüsenspezifische DNA-Sequenzen nachzuweisen. Hierbei kann es sich z.B. um Östrogen-Rezeptor-DNA, Mucin-Gen-DNA, eine brustdrüsenspezifische DNA-Sequenz im Zytokeratin-Gen oder um eine mutierte DNA handeln. Die mutierte DNA ist insbesondere eine DNA, die bei erhöhtem Brustkrebsrisiko oder bei manifestem Brustkrebs z.B. im Blutplasma auftritt. Dazu werden entsprechende Primer für den Östrogenrezeptor, für das Mucin-Gen, für die brustdrüsenspezifische Sequenzen im Zytokeratin-Gen sowie für die entsprechende mutierte DNA verwendet.

Durch das Verfahren wird es nun weiter möglich, den Hormonhaushalt von Frauen so einzustellen, daß die eumitotische Grenze nicht überschritten und somit das Brustkrebsrisiko drastisch verringert wird. Neben dem Nachweis von brustdrüsenspezifischen DNA-Sequenzen soll insbesondere in der zweiten Zyklushälfte auch nach Mutationen gefahndet werden, wie sie besonders häufig mit Brustkrebsrisiko vergeselischaftet sind. Es hat sich gezeigt, daß Zellen, in welchen Mutationen vorhanden sind, durch den Vorgang des natürlichen Zelltods (Apoptose) aus gesundem Gewebe eleminiert werden können. Solche durch Apoptose entstandene mutierte DNA kann im Plasma nachgewiesen werden. Lassen sich in der zweiten Zyklushälfte unter einer endogenen oder exogenen hormonalen Stimulation mutierte DNA-Bruchstücke im Plasma nachweisen, wie sie bei erhöhtem Krebsrisiko vorkommen können, so hat dies eine besondere Bedeutung für die weitere Diagnostik und Therapie.

Zusammenfassend ist festzustellen:
- Es konnte erstmalig gezeigt werden, daß brustdrüsenspezifische Gensequenzen unter einer Östrogen- respektive Östrogen/Gestagen-Stimulation von Brustdrüsengewebe vermehrt im zirkulierenden Plasma auftreten;
- Es konnte erstmals eindeutig die "eumitotische Grenze" mit einem Gentest für Plasma definiert werden;
- Sowohl unter natürlich-menstruierenden Bedingungen, als auch unter den Bedingungen einer Hormonapplikation darf die eumitotische Grenze nicht überschritten werden, um einen "Brustschutz" d.h. eine Brustkrebs-Prophylaxe zu erzielen.

Dieses Verfahrens kann auch zur Frühdiagnose bzw. Prophylaxe von Mammakarzinomen eingesetzt werden, da schon bei Frühformen von Brustkrebs unter einer hormonellen Stimulation (vermehrt) mutierte DNA im Plasma erscheinen wird. Dabei kann das Verhältnis der Konzentration an Brustdrüsen-spezifischen DNAs in der ersten Zyklushälfte zur entsprechenden Konzentration in der zweiten Zyklushälfte im Bereich von 1:100, insbesondere im Bereich von 1:50 liegen. Das vorstehend beschriebene Diagnoseverfahren kann dabei durch geeignete Primerwahl zur Frühdiagnose von Mammakarzinomen in der Weise durchgeführt werden, daß mutierte zirkulierende DNA im Plasma unter einer hormonalen Stimulation nachgewiesen wird.

### Beispiel:

Aus EDTA-Blut wird durch eine Zentrifugation bei 3000 upm Plasma gewonnen. Von dem klaren Überstand (Plasma) werden 900 µl entnommen und die darin enthaltende DNA aufgereinigt (Qiagen Blood-Kit). Die DNA wird in 50 µl Chargen von der Säule eluiert und eingesetzt.

10 µl der so gewonnenen DNA-Lösung werden direkt mit einem Polyacrylamid Gel auf die Konzentration untersucht (Vergleichskonzentrationsreihe mit einer DNA-Lösung bekannter Konzentration).

10 µl der Plasma-DNA-Lösung werden jeweils zu verschiedenen PCR-Reaktionen eingesetzt. Auf diese Weise kann die ursprüngliche DNA Konzentration noch einmal abgeschätzt werden und das genaue Verhältnis zu einer Plasma-DNA-Probe eines anderen Zyklustages bestimmt werden.

## Patentansprüche

1. Verfahren zur in vitro Diagnose des Proliferationsgrades der weiblichen Brust, dadurch gekennzeichnet, daß die Konzentration an zirkulierenden brustdrüsenspezifischen DNA-Sequenzen im Blutplasma der Frau bestimmt wird.

2. Verfahren nach Anspruch 1, dadurch gekennzeichnet, daß Blutplasma verwendet wird, das
a) einer normal menstruierenden Frau,
b) einer Frau, die Kontrazeptiva zuführt, oder
c) einer klimakterischen oder postmenopausalen Frau, die Hormone zuführt,
in der zweiten und gegebenenfalls auch in der ersten Zyklushälfte entnommen wird.

3. Verfahren nach einem der vorstehenden Ansprüche, dadurch gekennzeichnet, daß die Konzentrationen der brustdrüsenspezifischen DNA-Sequenzen in Blutplasma aus der ersten und zweiten Zyklushälfte verglichen werden.

4. Verfahren nach einem der vorstehenden Ansprüche, dadurch gekennzeichnet, daß die DNA
a) Östrogen-Rezeptor-DNA,
b) Mucin-Gen-DNA,
c) eine brustdrüsenspezifische DNA-Sequenz im Zytokeratin-Gen ist, oder
d) eine mutierte DNA ist.

5. Verfahren nach einem der vorstehenden Ansprüche, dadurch gekennzeichnet, daß die DNA-Sequenzen durch die Poymerase-Kettenreaktion oder andere Amplifikationsverfahren nachgewiesen werden.

6. Verfahren nach Anspruch 5, dadurch gekennzeichnet, daß spezielle Primer für
a) Östrogen-Rezeptor-DNA,
b) Mucin-Gen-DNA,
c) eine brustdrüsenspezifische DNA-Sequenz im Zytokeratin-Gen oder
d) mutierte DNA eingesetzt werden.

7. Verfahren nach einem der vorstehenden Ansprüche, dadurch gekennzeichnet, daß die eumitotische Grenze einer Hormongabe bei
a) kombinierter zyklischer kontrazeptiver Therapie
b) kontinuierlich-kombinierter kontrazeptiver Therapie, oder
c) Östrogen- oder Gestagenmonotherapie
bestimmt wird.

8. Verfahren nach einem der vorstehenden Ansprüche zur Diagnose des Brustkrebs-Risikos von Frauen.

9. Verfahren nach einem der Ansprüche 1 - 7 zur Diagnose von Brustkrebs bei Frauen.
